# EUROPEAN PATENT APPLICATION

(11) **EP 2 626 329 A1**
(43) Date of publication of application: **14.08.2013**
(21) Application number: 11830249.6
(22) Date of filing: 27.09.2011
(51) Int. Cl.: B82B 1/00, B82B 3/00, B82Y 5/00, B82Y 15/00, B82Y 40/00

(54) **METHOD FOR COATING AND FUNCTIONALIZING NANOPARTICLES BY MEANS OF A MICHAEL REACTION**

(30) Priority: 07.10.2010 ES 201031493 P
(71) Applicant: Consejo Superior De Investigaciones Científicas (CSIC), 28006 Madrid (ES)
(72) Inventor: PIÑOL LACAMBRA, Rafael, E-50009 Zaragoza (ES); MILLÁN ESCOLANO, Ángel, E-50009 Zaragoza (ES); PALACIO PARADA, Fernando, E-50009 Zaragoza (ES); GABILONDO UGARTE, Lierni, E-50009 Zaragoza (ES)
(74) Representative: Ungria López, Javier
(86) International application number: PCT/ES2011/070674
(87) International publication number: WO 2012/045902

(57) **Abstract**

The present invention relates to a method for coating nanoparticles to achieve stable dispersions of said particles in a liquid medium and the surface functionalization thereof with groups that have physical activity such as luminescence, chemical activity such as catalytic capacity and/or biological activity such as a capacity for selectively binding with a biological entity.

## Description

The present invention relates to a method for coating nanoparticles to achieve stable dispersions of said particles in a liquid medium and the surface functionalization thereof with groups that have physical activity such as luminescence, chemical activity such as catalytic capacity and/or biological activity such as a capacity for selectively binding with a biological entity.

### PRIOR ART

The applications of the nanoparticles extend to many areas in the industry because they offer numerous and diverse advantages with respect to microscopic and macroscopic materials. In structural materials such as ceramics and composites, they improve their mechanical performance. In functional materials, they offer unique characteristics and modulation capacity based on the fact that the physical properties of the materials (electric, magnetic, optical, chemical, biological, steric, etc.) change when their size enters in the nanometric range, and this variation takes place gradually with the size decrease. In their applications, the nanoparticles are available in the form of a mass, as films, as powders, as dispersions in a fluid or as aerosols. One of the first applications in the biomedical field was in the area of the biotechnology in the 1970s as carrier of enzymes and then in separation, purification, analysis, biocatalysis and bioprocessing (magnogel, dynabeads and estapor). In the 1980s, they started to be used as contrast agents in magnetic resonance imaging (MRI). And more recently, a large variety of uses in areas such as controlled administration of drugs, immunoassays, molecular biology, purification of nucleic acids DNA, cellular separation, therapy by means of hyperthermia, etc., have been described. As regards their industrial applications as suspensions of nanoparticles, their usefulness in the magnetic printing, magnetic inks, lubrication and sealing in vacuum systems, suspension systems, magnetic sensors, actuators, recuperation of metals, purification of water, etc., have been described. These applications are based on their large specific surface, on their capacity to cross biological barriers, their capacity for absorption of ions, and mainly on their exclusive magnetic, optical and electrical properties, which appear only at nanometric level, such as superparamagnetism, magnetoresistance, magnetic anisotropy, luminescence, etc.

There are various types of suspensions of organic and inorganic nanoparticles with technological applications. More recently, there is a great interest in the use of aqueous suspensions of magnetic nanoparticles and among them those of the iron oxides are very attractive due to their biocompatibility and their usefulness in biomedical applications (Laurent, S. Chem. Rev. 2008, 108, 2064). The first requirement in the manufacturing of these suspensions is to achieve their stability. A second point is the incorporation of one or several functionalities into the surface of the particles. For both things it is necessary to coat the nanoparticles from the adequate material. There are various strategies for the coating of nanoparticles, which can be classified depending on the type of the compounds which are used in the coating, as follows:
1) Coatings which contain silicon. This is the most widely used technique; it is based on the use of organo-silane precursors and consists in their adsorption in the surface and their subsequent hydrolysis in order to form layers of silicon or organo-silicon, which in turn can be activated by reacting with functionalized alkoxysilane compounds [WO2008/058678]. In [US6514481, Prasad], nanoparticles of iron oxide coated with silicon, to which a peptide is added by means of a spacer, are described; and in the patent application W02006/055447 a similar procedure for obtaining organo-silicon coatings functionalized with oligonucleotides is described. One system, which is used very much in the coating of particles, consists in hydrolysis of organo-silane precursors in the core of micelles [US2007110816].
2) Polymeric coatings. By using this technique, the coating is deposited by the combination of the nanoparticles with polymers soluble in the liquid medium and previously synthesized, which contain functional groups capable to interact with the surface of the particles in such a way that the particles become encapsulated in the interior of the polymer; or by adsorption of monomers or initiators of polymerization and subsequently by polymerization in situ, normally in the core of micellar systems. Polymers widely used in the coating of nanoparticles are the dextrane [US4452773], proteins [US2010/0029902], alginates; and synthetic polymers such as functionalized polystyrenes, polypyrrole, phenolic polymers, polymers of carboxylic acids [WO2005/112758], block polymers [Sohn et al. J. Appl. Polym. Sci. 2004, 91, 3549], polyethylenimines [W02009/135937] and others. One of the preferred techniques for increasing the stability of the coating consists in cross-linking the polymeric chains [W02003/005029]. Methods for coating nanoparticles with a double organic layer, where the union between the two layers takes place by means of amide linkages, have also been described [US2008/0226895]. The functionalization can be carried out by means of the physical absorption of a second polymer, such as in [US2003185757], in a first polymer, the carboxy dextran, used as stabilizer and a second polymer, carboxy dextran functionalized with enzymes, used for adding the functionality. A method, which permits the biocompatible coating and the functionalization of nanoparticles, consists in the use of the proteins barnase and barstar, but its high cost can limit its application [Nikitin et al. Proc. Nat. Acad. Sci. USA 2010 (/doi/10.1073/pnas. 1001142107)]. Methods for coating nanoparticles with polymers in aerosols [US2009/0252965] and in supercritical fluids [WO2004/091571] have also been described.
3) Another strategy uses compounds with low molecular weight and with groups which are capable to attach themselves to the surface of the particle connected to residues with affinity to the solvent. For example, in the patent WO 03/016217, metal nanoparticles are coated and, in particular, such of metal oxides with derivatives of phosphoric acid, phosphonic acid or phosphines. However, it is more customary to use as coating amphiphilic molecules which can be arranged in the form of simple or double layers such as, for example, in [Shen et al. Langmuir, 2000, 16, 9907], although in this case the suspensions are stable only at pH higher than 7.4.

On the other hand, mechanical methods for the coating of nanoparticles have also been described [WO2008/074087], although the control over the structure and thickness of the coating allowed by these techniques is not sufficient for some applications.

The functionalization of nanoparticles, i.e. the anchoring of active agents, is performed in the majority of the existing methods on the surface of the stabilizing layer, once the nanoparticle has been coated with this layer. This strategy has some inconveniencies such as processes of purification and limitations over the reaction conditions in order to avoid the aggregation or the deterioration of the stabilizing layer or the functionality to be added. The functionalization can be performed by means of electrostatic adsorption [Wang et al., IEEE Trans. Nanotech, 2009, 8, 142], but it is preferable to do it by means of covalent bonds, through a spacer which connects the surface of the particle with the active agent [Georgelin et al., J Nanopart Res 2010, 12. 675]. One versatile method is based on the specific bonding between the streptavidin and the biotin, although its cost is elevated and requires the use of biotinated active agents.

On the other hand, the Michael reaction has been used in the preparation of massive materials, such as in the hydrophilization of the surfaces of polymeric objects, in special contact lenses [US2008/0003259], in the manufacturing of hydrogels, in the curing of silanes [WO2006/087079], in the manufacturing of laminated materials, in the synthesis of pharmaceutical products [US2008/0213249], and in the production of lacquers and adhesives.

### DESCRIPTION OF THE INVENTION

The present invention provides a method for obtaining coated and functionalized nanoparticles, the use of the said particles obtained by said method, to form stable dispersions in a liquid medium; it provides also the stable dispersions and, in addition, the use of these dispersions in biomedicine.

The method of the invention is based on the use of the Michael reaction. In this invention, the Michael addition reaction is used for coating nanoparticles with layers solvatable by a liquid, so that they will be stable in suspension in said liquid and add active agents in the surface of the said nanoparticles.

One first aspect of the present invention is related to a method for obtaining nanoparticles coated and functionalized by means of the Michael reaction, which comprises the steps:
a) reaction of at least one nanoparticle with a reagent comprising a Michael donor (B) or acceptor (C), and
b) reaction of the product obtained in (a) with a reagent which comprises a Michael acceptor (C) or donor (B).

If in the step (a) the reagent is a donor (B), in the step (b) the reagent is an acceptor (C) or vice versa.

The term "Michael reaction" in the present invention comprises the concepts of Michael reaction, Michael addition, and Michael type reaction, which are known by any person skilled in the art. Therefore, in the present invention, "Michael reaction" is understood as the addition 1, 4 of a carbanion stabilized through resonance or nitrogen, sulfur or oxygen nucleophiles (Michael donor B) to an unsaturated system conjugated with an attracting group of electrons (Michael acceptor C).

In the present invention, "nanoparticles" are understood as organic, inorganic or hybrid organo-inorganic particles with a size between 1 nm and 100 nm.

Preferably, the nanoparticle is selected from metal, metal oxide or any of their combinations, but, preferably, the metal oxide is an iron oxide.

In one preferred embodiment, the reagent of the step (a) comprises additionally a spacer R₁, in a more preferable embodiment R₁ is an organic component.

In another preferred embodiment, the reagent of the step (b) comprises additionally a spacer R₂. In a more preferable embodiment, R₂ is an organic component which comprises groups solvatables in a liquid.

Preferably, the reagent of the step (a) of the invention comprises additionally a bonding group (A) selected from a cation, an anion, alkoxylane precursor, thiol, alcohol, alcoxide, carboxylate, carboxylic anhydride, phosphate, polyelectrolyte, imine, nitrile, azide, amine, amide, phosphine or any combination thereof. More preferably, the cation is a quaternary amine. And more preferably, the anion is selected from a carboxylate, sulfate, phosphate group or any combination thereof. This bonding group (A) ensures the direct bonding to the nanoparticle, so that it does not remain simply physically absorbed, conferring a greater stability to it.

In one preferred embodiment, the Michael donor reagent (B) is selected from the list comprising: β-diketone, malonic ester, β-ketoester, β-ketonotrile, nitro, amino, malonic acid, enamine, primary amine, secondary amine, tertiary amine, imine, hydracine, guanadine, alcohol, thiol, phosphine, methylene, carbinol, organo-metallic compound, halide, and any combination thereof.

In one preferred embodiment, the Michael acceptor reagent (C) is selected from the list comprising: conjugated enone, carbonyl, cyan, carboxylic, carboxylate, sulfonil, aldehyde, ester, nitrile, unsaturated nitro, carboxylate, acrylate, acrylamide, methacrylate, methacrylamide, acrylonitrile, vinyl ketone, vinyl sulfone and any combination thereof.

Preferably, the reagent of step (b) of the method according to the invention comprises additionally at least one organic or inorganic residue (D) with physical, chemical or biological functionality.

More preferably, the organic or inorganic residue (D) with physical functionality is selected from fluorescein, rhodamine, rare earth complex, quantum dots or any combination thereof.

More preferably, the organic or inorganic residue (D) with chemical functionality is selected from a catalyst or an absorbent. And even more preferably, the catalyst is a coordination compound of rhodium.

In one more preferable embodiment, the organic or inorganic residue (D) with biological functionality is selected from a compound with catalytic capacity, with recognizing capacity, with curative capacity or any combination thereof. In one even more preferable embodiment, the compound with catalytic capacity is an enzyme. The component with recognizing capacity can be selected from antibodies, oligonucleotides or any combination thereof. And in another even more preferable embodiment, the compound with curative capacity is a medicine.

Preferably, the steps (a) and/or (b) take place at temperature conditions between 10 and 70°C.

The method proposed in this invention offers various advantages compared to the preexisting methods, which as a whole represents a great advance in the state of the art.

The present invention permits the stabilization of nanoparticles in a liquid medium by means of coating the surface of the nanoparticles with residues solvatable in this medium. The bonding of solvatable residues to the surface of the particles is produced by means of covalent bonds, which ensures the great stability of the coating. In addition, these residues can perform other functionality than the mere stabilization, such as the control over the surface reactivity of the nanoparticle. This concept is exemplified, without limiting the scope of this invention, with the utilization of the coatings containing residues of polyethylene glycol, PEG, which, in addition to allow the stabilization of the nanoparticles in water, reduces the adherence of proteins to the particles.

As regards the functionalization, the method of the invention is a real synthetic platform for nanoparticles since it permits to add various physical, chemical and biological functionalities to the same nanoparticle.

Another advantage consists in the fact that it permits a high density of the coating, which confers a greater stability to the suspension.

Another advantage in the state of the art, which this invention brings, is the capacity to control the quantity of functional groups in the surface of each particle in a simple way by the use of different proportions of functionalized and non-functionalized residues.

Another great advantage compared to other techniques, which permit the anchoring of functionalities by means of covalent bonds, is that in this invention the anchoring reaction is performed in very smooth conditions, which permits to perform functionalizations with easily degradable active agents.

Another great advantage compared to the methods of functionalization based on biological compounds, is the simplicity of the application.

A second aspect of the present invention is related to the coated and functionalized nanoparticle which can be obtained by the method of the invention.

A third aspect of the present invention is related to the use of the above-described nanoparticles for the manufacturing of products which are selected from the list comprising cosmetics, paints, inks, catalysis, purification of water and biomedicine. This is so because said nanoparticles can form stable dispersions.

The method of the invention permits the functionalization of a particle, which has already been stabilized with a protective layer, but it goes further by permitting the bonding of the functional molecule to the residue which has formed the protective layer prior to the coating. In this way, the purification is facilitated and the restrictions to the reaction conditions, which are imposed by the stability of the suspension, are eliminated.

Another aspect of the present invention is related to a dispersion of the coated and functionalized nanoparticles, as described above, in a liquid medium, which is called also mother liquid.

In the present intention "mother liquid" is understood as a solution in a solvent of an aqueous or organic type, which can contain or not contain additives such as ionic, neutral or anfifilic compounds. The mother liquid can be water or a physiological liquid.

In a preferred embodiment, the liquid dispersion medium is selected from aqueous medium, organic medium or any combination thereof.

The method of the invention permits the preparation of dispersions called ferrofluids, which are understood as stables suspensions of magnetic nanoparticles. In one more preferable embodiment of this invention, the nanoparticles are from magnetic iron oxide and have superparamagnetic properties, as it is shown in Example 6.

At the same time, the method of the invention permits the preparation of suspensions of multifunctional nanoparticles.

Another aspect of the present invention is related to the use of the above-described dispersion for the manufacturing of products which are selected from the list comprising cosmetics, paints, inks, catalysts, water purification and biomedicine.

In the course of the description and the claims, the word "comprise" and its variants do not intend to exclude other technical characteristics, additives, components or steps. For the person skilled on the art, other objects, advantages and characteristics of the invention are deduced partially from the description and partially from the practice of the invention. The following examples and drawings are provides as an illustration and they are not limitative of the present invention.

### DESCRIPTION OF THE FIGURES

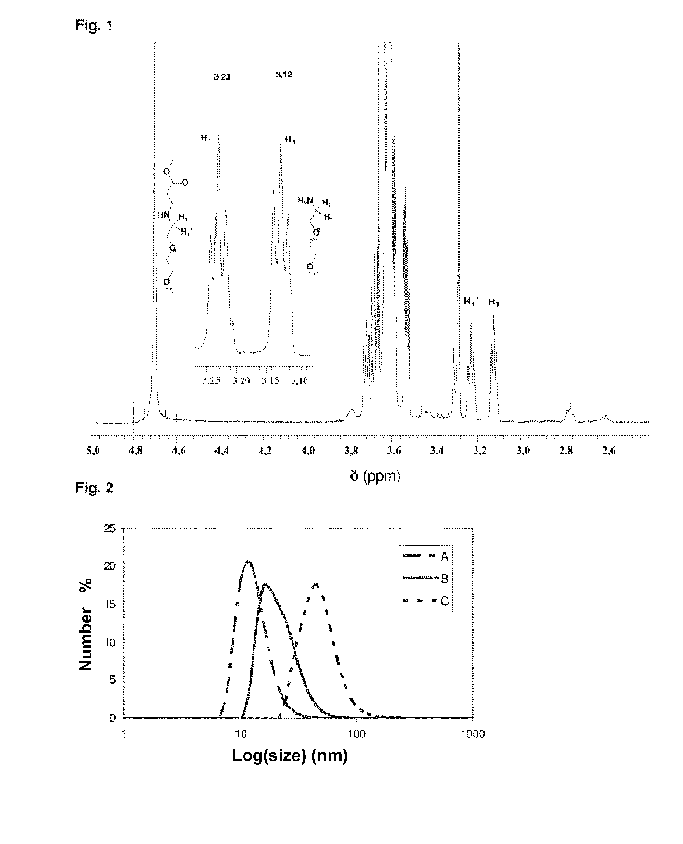

**Fig. 1.** ¹H-RMN (D₂O, 400 MHz). Spectrum corresponding to the final product obtained according to example 1. In the top left part, an enlargement of the zone of the spectrum corresponding to 3.07-3.27 ppm is shown. The triplet observed at 3.12 ppm belongs to the protons marked as H₁ in the figure (CH₂-CH₂-NH₂) and which correspond to the starting amine MeOPEG₃₅₀-NH₂. The triplet observed at 3.23 ppm is assigned to the protons marked as H₁' in the figure (-CH₂-CH₂-NH-CH₂-CH₂-C(=O)OCH₃) and which correspond to the product of the reaction between the amino-group of MeOPEG₃₅₀-NH₂ and the methyl acrylate by means of addition of Michael type. The relation of the intensities between the two signals indicates a conversion of 50%.
**Fig. 2****.** Distribution of the hydrodynamic diameters of nanoparticles suspensions prior to the coating (FFa) manufactured according to example 2, after the coating with acrylic acid (FFa@Acr) according to example 3, and after a second coating with n-amino polyethylene glycol (FFa@Acr@PEG-NH2) according to example 3.

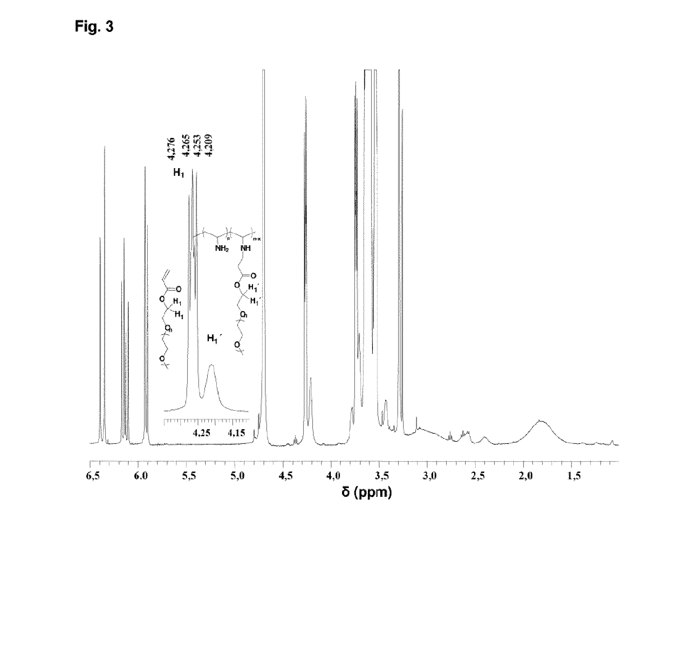

**Fig. 3****.** ¹H-RMN (D₂O, 400 MHz). Spectrum corresponding to a sample manufactured according to example 4, extracted after 20 hours of reaction at room temperature. In the top left part, an enlargement of the zone of the spectrum corresponding to 4-4.35 ppm is shown. The triplet observed at 4.27 ppm belongs to the protons marked as H₁ in the figure (CH₂=CH-(O=C)-O-CH₂-CH₂-) and which correspond to the starting acrylate MPEGA₄₅₄. The wide singlet observed at 4.21 ppm is assigned to the protons marked as H₁' in the figure (-NH-CH₂-CH₂-(O=C)-O-CH₂-CH₂-) and which correspond to the product of the reaction between the amino-group of the polymer and the starting acrylate by addition of Michael type. The relation of the intensities between the two signals indicates a conversion of 27%.

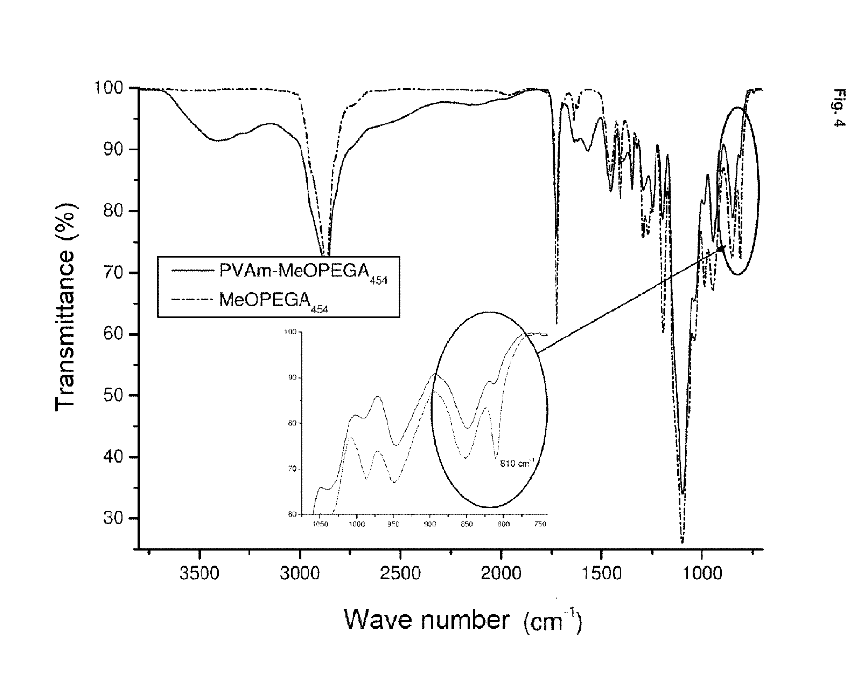

**Fig. 4****.** The figure shows the FT-IR spectrum corresponding to the product obtained by the Michael addition between PVam and MeOPEGA₄₅₄, manufactured according to example 4 after 20 hours of reaction (continuous line), and the spectrum of the starting product MeOPEGA₄₅₄ (discontinued line). In the left bottom corner, an enlargement is presented of the zone corresponding to 1050-750 cm⁻¹. In the enlargement, one can observe the disappearing of the band at 810 cm⁻¹, which corresponds to the vibration of the flexion (outside of the plane) of the vinylic group in the spectrum of the final product and present in the spectrum of the starting product. The absence of this band confirms the reaction of addition between the amino-groups of the polymer and the acrylate.

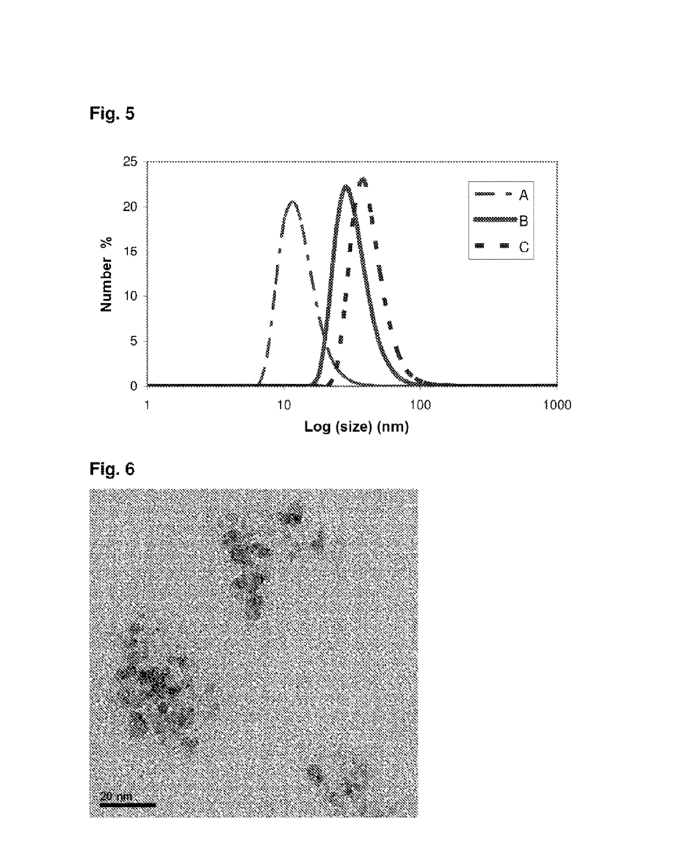

**Fig. 5****.** Distribution of the hydrodynamic diameters of the nanoparticles suspensions prior to the coating (FFa) manufactured according to example 2, after the coating with polyvinylamine (FFa@PVAm) according to example 5, and after a second coating with polyethylene glycol acrylate (FFa@PVAm@PEG-A) according to example 5.
**Fig. 6****.** Images TEM of the nanoparticles suspension coated with polyvinylamine (FFa@PVAm) according to example 6, and after a second coating with polyethylene glycol acrylate methylfluorescein (FFa@PVAmMeFluPEG-A) in a physiological tampon at pH=7.40 (PBS) manufactured in example 6.

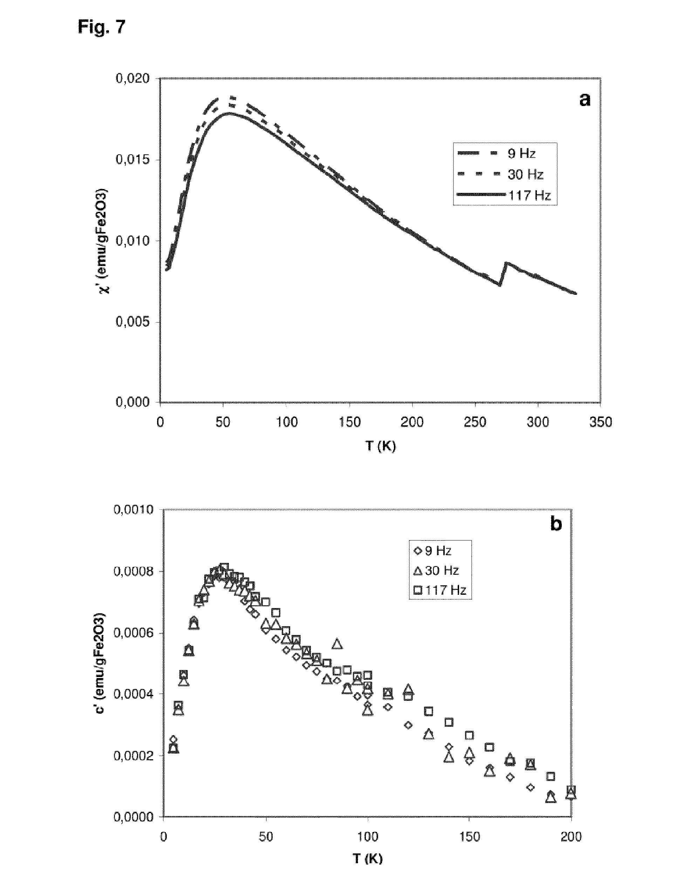

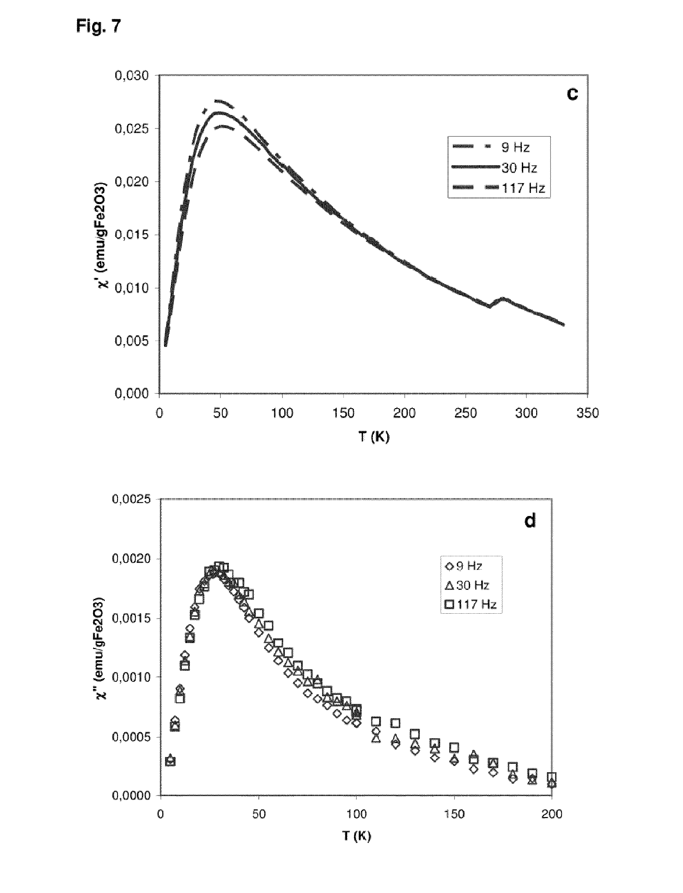

**Fig. 7****.** Variation of the susceptibility ac, a) in phase (χ'), b) out of phase (χ") in alternate magnetic field of nanoparticle suspensions prior to the coating (FFa) and c) and d) after coating with polyethylene glycol acrylate methylfluorescein (FFA@PVAmMeFluPEG-A) in a physiological tampon at pH=7.40 (PBS) manufactured in example 6.

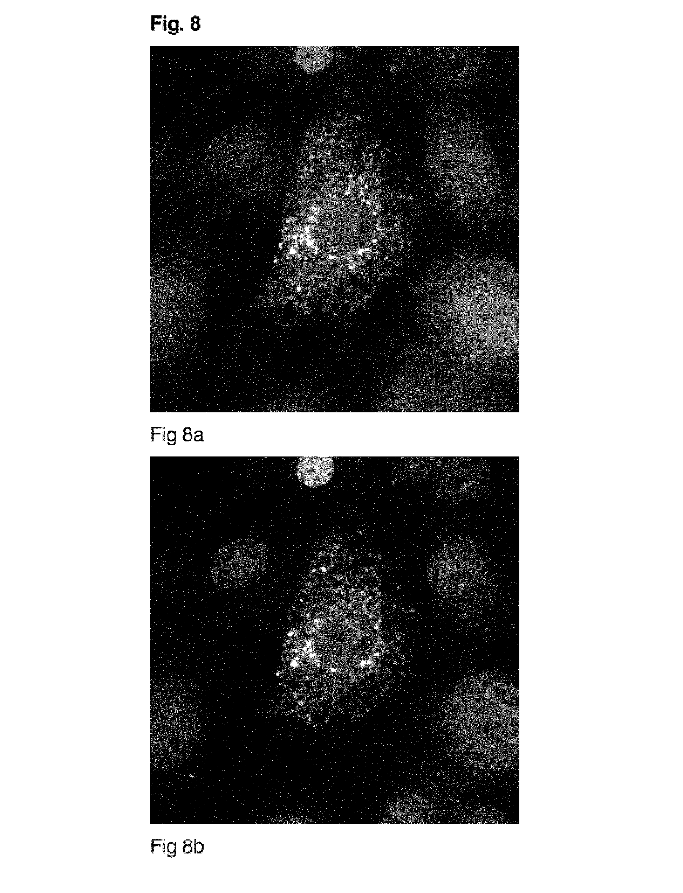

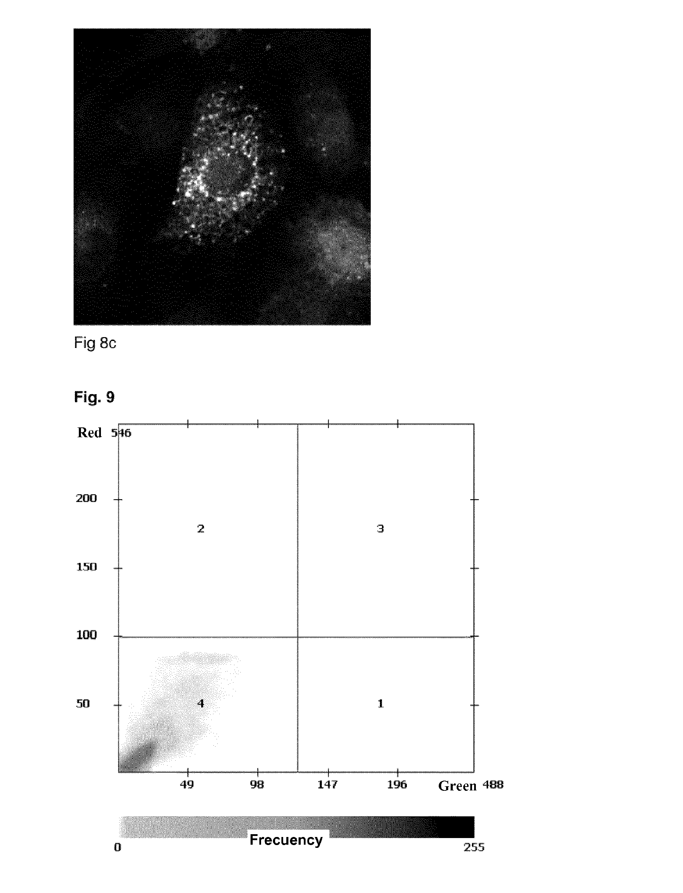

**Fig. 8****.** Optical microscopy images of fluorescence obtained by the technique of structured imaging of a cell of the smooth muscle of the aorta of a rat in a medium containing the ferrofluid FFa@PVAmMeFluPEG-A; (a) the glittering points correspond to the functionalized nanoparticles and appear in green in the color image, (b) the glittering points correspond to the lysosomes marked with a lysotracker (molecular probes-invitrogen) and appear in red in the color image, (c) a mixed image, in which the glittering points, of greater intensity than the images (a) and (b), which would correspond to the yellow-orange coloration in the color image, coincide in the same visual field of the internalized nanoparticles and of the lysosomes shown in the images (a) and (b), respectively. The nuclei appear in blue in the images.
**Fig. 9****.** Graphics of the dispersion of the emissions of the functionalized nanoparticles, axis x, and of the lysosomes marked with a fluorescent substance, axis y, in the cell culture whose image is shown in figure 8.

### EXAMPLES

Here below the invention will be illustrated by some experiments conducted by the inventors, which demonstrate the specificity and effectiveness of the method of the invention for the synthesis of coated and functionalized nanoparticles, such as the dispersion in a liquid medium of the said nanoparticles.

### Example 1

In this example, it is illustrated how a reagent X, which contains an anchoring group or bond A, in this case a carboxylate group, and one Michael acceptor C, in this case a double bond conjugated with carboxylate, is able to react by means of a Michael addition with a reagent Y, which contains a Michael donor B, in this case a -NH₂ group, and a hydrophilic residue, in this case polyethylene glycol, at room temperature and under soft pH conditions, close to the physiological pH, pH: 7.53, with yield of at least 50%.

### Synthesis of MeOPEG₃₅₀OTs

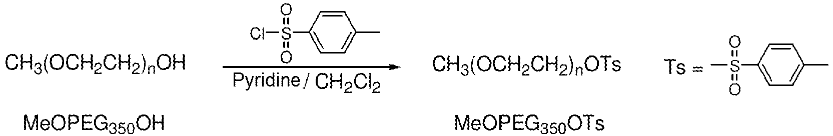

In a 250 ml three-neck flask loaded with a magnetic stirrer, 120 ml anhydrous dichloromethane, 21.02 g methoxy polyethylene glycol (MeOPEG₃₅₀OH, Mn: 350 Da, Aldrich, 60 mmol) and 9.7 ml pyridine (120 mmol) are added. Under argon atmosphere and with agitation, 23.34 g tosyl chloride (120 mmol) are added slowly to the obtained solution. The mixture is maintained in agitation at room temperature for 70 hours, it is filtered and is poured over 60 ml cold distilled water. The organic phase is separated and is washed successively with 60 ml of aqueous solution of pyridine at 25% v/v, with 60 ml cold distilled water, after that with two 60 ml fractions of cold hydrochloric acid 6N, and finally with two 60 ml fractions of cold distilled water. The organic phase is dried over anhydrous magnesium sulfate, is filtered and finally the solvent is evaporated in the rotary evaporator obtaining 26.46 g (52.2 mmol) colorless oil is obtained. Yield: 88%.

### Synthesis of MeOPEG₃₅₀-NH₂.

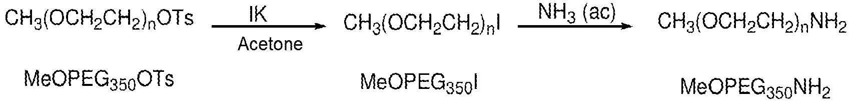

In a 250 ml three-neck flask loaded with a mechanic stirrer and equipped with a refrigerant, 17.76 g potassium iodide (107 mmol) are added to a solution of 25.21 g methoxy polyethylene glycol tosyl (MeOPEG₃₅₀OTs, 50 mmol), obtained in example 1, in 90 ml acetone. The mixture is heated to reflux (70° C) for 24 hours. After cooling off at room temperature, it is filtered and the solvent evaporated in the rotary evaporator obtaining viscous yellow oil. The product is used in the next step without additional purification. In a 1 L two-neck flask loaded with a magnetic stirrer, the methoxy polyethylene glycol (MeOPEG₃₅₀I), obtained in the previous step (approx. 50 mmol) and dissolved in 25 ml distilled water, is added by means of a funnel drop by drop to 375 ml aqueous ammonia (25%, 6 mol) under argon atmosphere. After 5 days stirring at room temperature, the reaction is considered finished. The product of the reaction is extracted with 3 fractions of 125 ml dichloromethane, the combined organic phases are filtered through paper, dried on anhydrous magnesium sulfate and the solvent is evaporated in the rotary evaporator, thus obtaining 19.05 g yellowish oil. The crude oil is dissolved in 50 ml diethyl ether, after the addition of 50 ml n-hexane and stirring the mixture is stored in the freezer. On the next day, the mixture is separated by means of decanting process, separating the oil precipitated on the bottom of the flask and the supernatant solvent. After the evaporation of the supernatant solvent, 10.83 g (31 mmol) colorless oil are obtained.

### Reaction of acrylate and MeOPEG₃₅₀-NH₂ by means of Michael addition

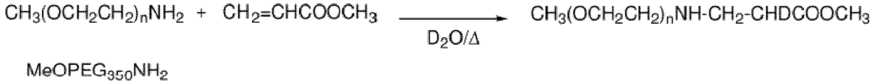

In a 20 ml one-neck flask, 316 mg MeOPEG₃₅₀NH₂ (0.9 mmol), obtained in example 2, are dissolved in 5 ml D₂O, the pD of the solution is adjusted to 7.53 by adding DCI diluted with D₂O in a ratio of 1:20 v/v. 81 µl methyl acrylate (Aldrich, 0.9 mmol) are added to the colorless and transparent solution and the mixture is stirred for 88 h at room temperature. The obtained solution is analyzed by means of proton nuclear magnetic resonance (¹H-RMN) by calculating a conversion of 50% (Figure 1). The FT-IR of the solid obtained after washing the aqueous phase with 10 ml dichloromethane and evaporating in the rotary evaporator, confirms that the obtained product corresponds to the product resulting from the Michael addition type of the amino-groups of the polymer with the acrylate.

### Example 2. Synthesis of iron oxide nanoparticles

In this example, a dispersion of nanoparticles in aqueous medium is prepared. The iron oxide nanoparticles are prepared by basic hydrolysis of a solution of FeCl₂/FeCl₃ in water following the procedure described in [Kang et al, Chem. Mater. 1996, 8, 2209] with some modifications, using reagents supplied by Aldrich. 250 ml of a solution of NaOH 1.5 M are added drop by drop under magnetic stirring to 25 ml of a solution which contains 3.137 g FeCl₂.4H₂O, 5.200 g FeCl₃ and 0.85 ml conc. HCl. The obtained suspension is maintained under agitation for 30 min and the precipitate is separated by magnetic decantation. The precipitate is washed two times by means of dispersion in water, centrifugation and redispersion; and then it is dispersed finally in 300 ml of a solution of HCl 0.01 M in order to obtain a stable suspension with pH=1.6 and concentration of 1.74 g/I in Fe₂O₃, designated as FFa. The distribution of the hydrodynamic sizes, measured by dynamic dispersion of light (DLS) with a Nanosizer ZS from Malvern device, is shown in figure 2. The average hydrodynamic diameter is 13 nm.

### Example 3

This example illustrates the coating of nanoparticles, dispersed in water, with hydrophilic residues by the addition of Michael and the obtaining of stable suspensions of nanoparticles at physiological pH (pH = 7.4) by means of a process which includes the coating of the particles with a Michael acceptor and a subsequent reaction with a Michael donor containing hydrophilic residues.

### Coating of nanoparticles with acrylate and MeOPEG₃₅₀-NH₂ and by addition of Michael

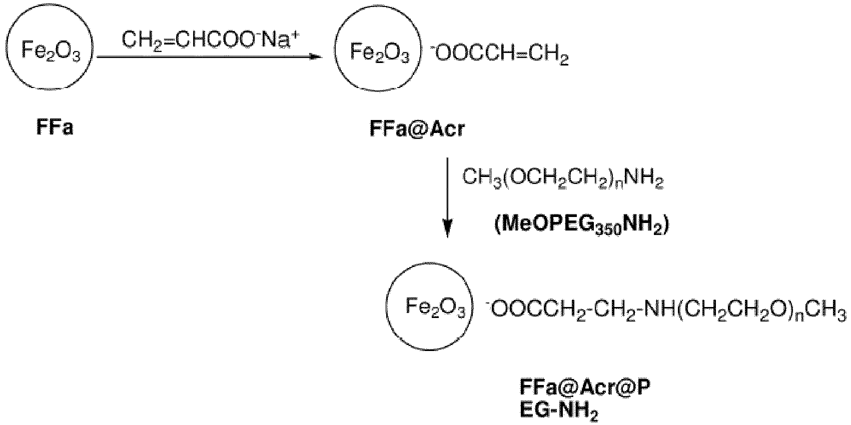

72.71 mg sodium acrylate (CH₂=CHCOO⁻Na⁺, 0.75 mmol) are added with agitation to 10 ml of the **FFa** synthesized in example 2. After the mixture is stirred for 30 minutes, a suspension of nanoparticles with pH: 4.17 denominated as ferrofluid **FFa@Acr,** is obtained. 262 mg amino-methoxy polyethylene glycol (MeOPEG₃₅₀NH₂, Mn: 349, 0.75 mmol) are added to this suspension, as a result of which the pH of the suspension reaches the value of 8.90. After stirring for 30 minutes, the pH of the suspension is adjusted to pH: 7.42 by slowly adding HCl 0.25 M. The suspension is maintained in agitation at room temperature for 1 hour, is subjected to ultrasound for 4 minutes and is filtered through a filter Milex®-GS MF (Milipore) of 0.22 µm in order to obtain a suspension of nanoparticles denominated as ferrofluid **FFa@Acr@PEG-NH2.** Figure 2 shows the distributions of the hydrodynamic diameters of the ferrofluids **FFa@Acr** and **FFa@Acr@PEG-NH2** measured by dynamic dispersion of light (DLS) with a Nanosizer ZS from Malvern device. It is observed clearly a regular displacement of the distribution of the sizes when passing from the **FFa** to **FFa@Acr** and then to **FFa@Acr@PEG-NH2,** which demonstrates the performing of the coating over the entire population of particles in both cases.

### Example 4

Contrary to example 1, this example illustrates how a reagent Y, which contains an anchoring group A, in this case an amino-group, and a Michael donor B, the amino-group itself, is able to react through the Michael addition with a reagent X, which contains a Michael acceptor C, in this case a double bond conjugated with a carboxylate group, and a hydrophilic residue, in this case polyethylene glycol, at room temperature and under soft pH conditions, close to a physiological pH - pH: 7.5, with a yield of at least 27%.

### Synthesis by radical pathway of poly N-vinylformamide ([(HCONH)CHCH₂]ₙ). PNVF.

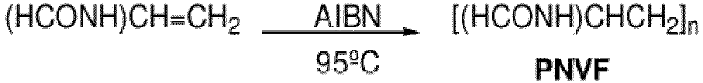

A mixture of 4 ml distilled N-vinylformamide ((HCONH)CH=CH₂, 57.06 mmol) and 50 ml 2-propanol is put in a 100 ml two-neck flask equipped with refrigerant and mechanical stirrer. The mixture is purged burbling argon through the dissolution during at least 15 minutes. After adding 187.40 mg of 2.2' azobisisobutyronitrile (AIBN, 1.14 mmol) under atmosphere of argon, the solution is introduced in an oil bath thermostated at 95° C. The mixture is stirred at 95° C for 1 hour under atmosphere of argon. After the flask is opened to the atmosphere, the mixture is cooled off to room temperature. The reaction mixture is filtered through a plate of porous glass and the obtained white solid is washed successively with 2 fractions of 25 ml cold 2-propanol and 25 ml acetone. The obtained solid is dried under vacuum at 50° C, obtaining 3.47 g **PNVF ([(HCONH)CHCH₂]ₙ).** Yield: 86%.

### Hydrolysis of PNVF. Synthesis of polyvinylamine ([(HCONH)CHCH₂]ₙ). PVAm

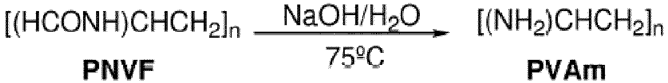

25 ml of a solution of sodium hydroxide 2M (50 mmol) are added to a mixture of 3.20 g **PNVF** (40 mmol, -NH-COH) in 30 ml distilled water. The resulting solution is stirred at 75° C for 60 hours. The solution is concentrated in a rotary evaporator up to a volume of approximately 20 ml and the obtained polymer is precipitated in excess of acetone (250 ml) at room temperature. The polymer is dissolved in 120 ml distilled water and is purified by dialysis against distilled water in the course of 3 days by using a Spectra/Por® membrane with a through cut of 1000 daltons. Finally, the solvent is evaporated in the rotary evaporator and the polymer is dried under vacuum, obtaining 1.47 g PVAm. Yield: 85%.

### Reaction of PVAm with MeOPEGA₄₅₄ by the Michael addition

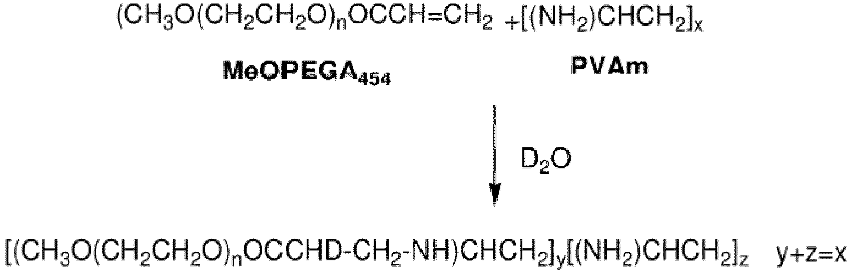

In a 20 ml one-neck flask, 53.84 mg PVAm (1.25 mmol) are dissolved in 10 ml D₂O. After the polymer is dissolved completely, the pD of the solution (pD: 10.57) is adjusted to pD: 7.53 by adding diluted DCI (1:20 v/v DCI (c)/D₂O) drop by drop and with agitation. 0.57 g methoxy polyethylene glycol acrylate MeOPEGA₄₅₄ (Aldrich, Mn: 454Da, 1.25 mmol) are added to the colorless and transparent solution. After stirring for 5 minutes at room temperature, a sample is taken and is analyzed by proton nuclear magnetic resonance (¹H-RMN), calculating a conversion of 10%. The solution is maintained in agitation at room temperature taking samples at different times and analyzing the progress of the reaction by means of ¹H-RMN. After 20 hours (see figure 3), a conversion close to 27% is obtained. The FT-IR of the solid obtained after washing the aqueous phase with 10 ml dichloromethane and evaporating in the rotary evaporator confirms that the obtained product corresponds to the product resulting from the the Michael addition type of the amino-groups of the polymer with the acrylate (see figure 4).

### Example 5

Contrary to example 3, this example illustrates the coating of nanoparticles dispersed in water with hydrophilic residues by means of a Michael addition and the obtaining of suspensions of nanoparticles stables at physiological pH (pH=7.4) through a process which includes the coating of the particles, first with a Michael donor, and then by reaction with a Michael acceptor which contains hydrophilic residues.

### Purification of PEGA₂₀₀. CH₂=CHCO(OCH₂CH₂)ₙOH

15 ml polyethylene glycol acrylate (Dajac Polymers, Mn: 245 Da), previously filtrated through a porous plate, are dissolved in 45 ml distilled water, obtaining a yellowish solution. The aqueous phase is extracted with 5 fractions of 30 ml diethyl ether. The aqueous phase is subsequently extracted with 2 fractions of 60 ml of a mixture of dichloromethane/n-hexane (3:1). The combined organic phases are filtered through a paper filter and after drying with anhydrous magnesium sulfate the solvent is evaporated in a rotary evaporator, obtaining 5.56 g oil with dark-yellow color.

### Coating of nanoparticles with PVAm and PEGA₂₀₀ by means of Michael addition

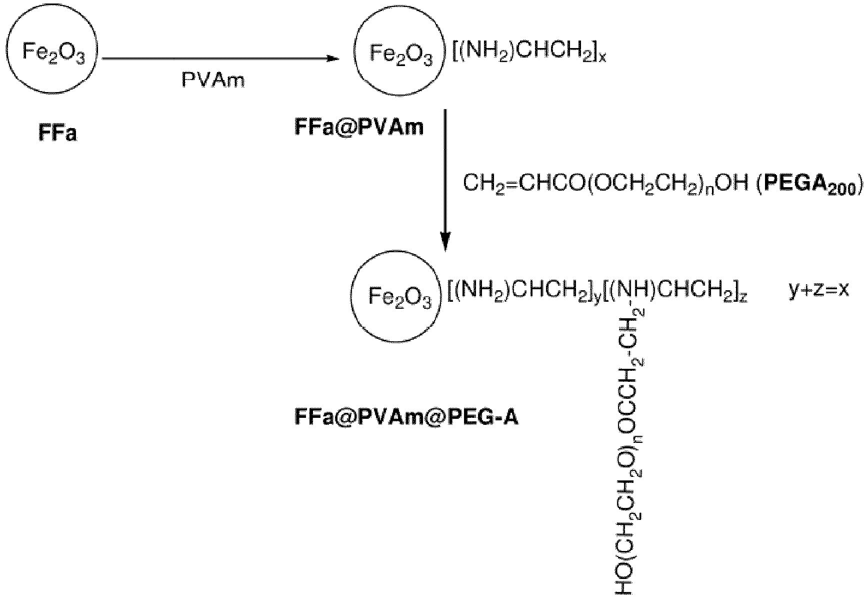

32.3 mg polyvinylamine (PVAm, 0.75 mmol -NH₂) are added with agitation to 10 mL of the FFa synthesized in example 2. After the complete dissolution of the polymer, a dispersion of nanoparticles is obtained, denominated as ferrofluid **FFA@PVAm,** with pH: 2.62. 190 mg of previously purified PEGA₂₀₀ (Mn: 248; 0.77 mmol) are added to this dispersion. The pH of the obtained suspension is slowly increased by adding a solution of NaOH 0.5 M drop by drop and with agitation. After reaching pH: 6.89, the mixture is adjusted at pH: 7.35 by adding Na₂HPO₄ 0.1 M. The suspension is stirred for 30 minutes at room temperature and finally is subjected to ultrasound for 4 minutes and is filtered through membrane Millex®-GS MF (Millipore) of 0.22 µm, resulting in a transparent suspension denominated as ferrofluid **FFa@PVAm@PEGA.** The distributions of the hydrodynamic sizes of the particles, measured by DLS after they are coated with PVAm and subsequently coated with PEGA, are shown in Fig. 5. The hydrodynamic diameters are 33 nm and 43 nm. The increase of the size compared to the initial ferrofluid (13 nm) indicates that the first coating with PVAm and the second coating with PEGA by means of the Michael reaction have been attained.

### Example 6

This example illustrates the coating of nanoparticles with hydrophilic residues, their functionalization with fluorescent inks, their superparamagnetic properties and their utility in biomedical studies, in particular, in the follow-up of processes of endocytosis.

### Synthesis of methylefluorescein (MeFluOH)

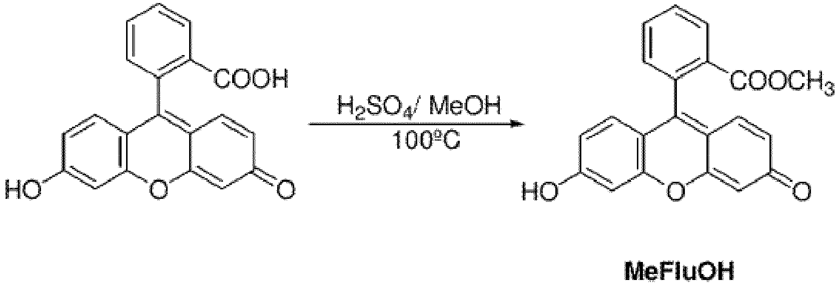

18.8 ml concentrated sulfuric acid are carefully added to a suspension of 25 g fluoresceine in 75 ml methanol cooled down in a water-ice bath. The solution with dark-red color is heated to reflux (100° C) during 15 hours. The suspension is cooled down to room temperature and 25 ml very cold water are added in a 500 ml beaker. 75 g sodium bicarbonate are added carefully and with strong agitation to the previous mixture. The mixture is filtered through a porous plate and the obtained solid is washed with 150 ml distilled water. The solid is re-suspended in 600 ml of an aqueous solution of sodium bicarbonate (2 weight %); after stirring for 15 minutes, it is filtered through a porous plate and is washed with 150 g distilled water. The process of washing with bicarbonate/water is repeated again by using the same volumes. Finally, the solid is re-suspended in 300 ml of an aqueous solution of acetic acid (1%); after stirring for 15 minutes, it is recuperated by filtration through a porous plate and the obtained solid is washed with 150 ml water. The obtained reddish solid is dried in the oven for two hours at 125° C. After drying, 14.87 g solid with orange color (42.9 mmol) are obtained. Yield: 57%.

### Synthesis of methyl fluoresceine polyethylene glycol acrylate (MeFluPEGA₂₀₀)

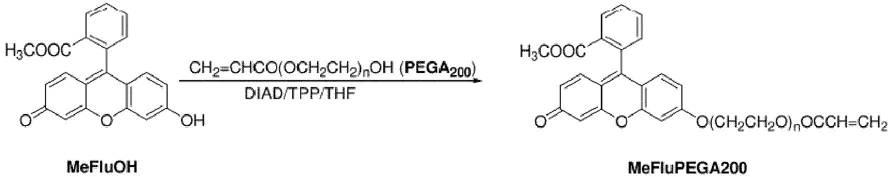

2.89 g triphenylphosphine **TPP** (11 mmol) and 2.75 g polyethylene glycol acrylate **PEGA**₂₀₀(~11 mmol, Mn: 248.27 Da), previously purified by extraction, are added to a suspension of 3.46 g **MeFluOH** (10 mmol) in 50 ml tetrahydrofuran anhydrous under atmosphere of argon. After cooling off the mixture in a water-ice bath, 2.3 ml diisopropyl-azodicarboxylate **DIAD** (11 mmol) are added with a syringe drop by drop under atmosphere of argon. Once the addition is finished and after stirring at 0° C for 15-20 minutes, the water-ice bath is removed and the mixture is maintained in agitation at room temperature. After stirring for 66 hours, 0.25 ml distilled water are added to the mixture and after stirring for 25 minutes the mixture is filtered through paper. The solvent is eliminated in the rotary evaporator and the obtained crude substance is dissolved in 50 ml dichloromethane. The organic phase is washed successively with 3 fractions of 25 ml of potassium carbonate 1 M, 25 ml of a saturated solution of sodium chloride, it is filtered through paper, dried on anhydrous magnesium sulfate and the solvent is evaporated in the rotary evaporator. The crude substance is purified two times by dissolution in a small volume of tetrahydrofurane (1 ml x g of crude substance) and by precipitation in excess of cold diethyl ether (25 ml x g of the crude substance). The product is purified by means of pressure chromatography on silicon gel by using initially ethyl acetate as eluyent and increasing progressively the polarity of the eluyent until terminating with a mixture of ethyl/methanol acetate (9.5:0.5). Yield: 2.40 g of the product were obtained.

### Coating and functionalization of nanoparticles with PVAm, PEGA₂₀₀ and MeFluPEGA₂₀₀ by means of Michael addition

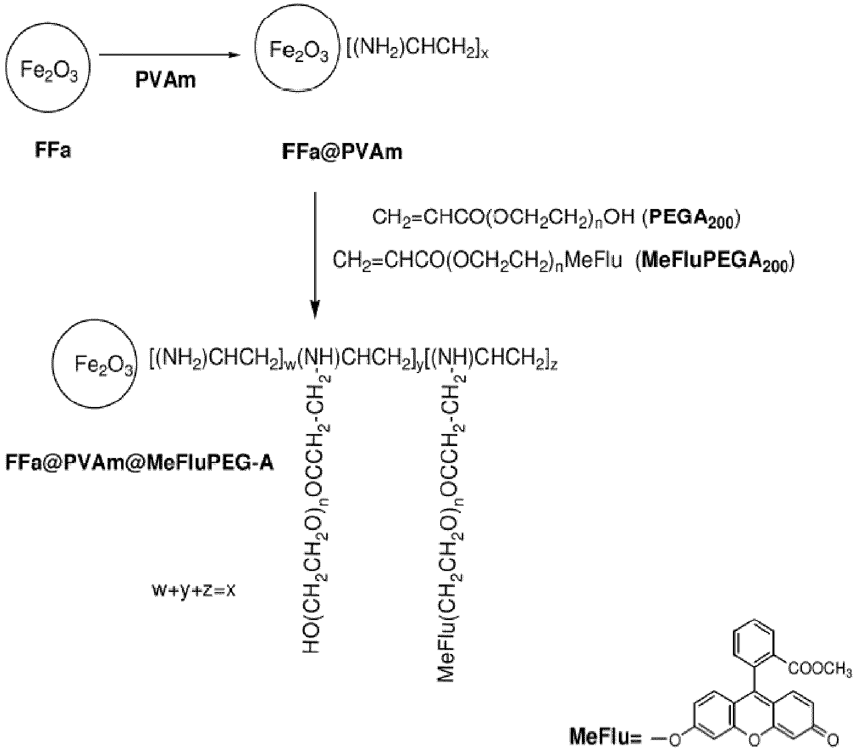

25.90 mg polyvinylamine (PVAm, 0.60 mmol -NH₂) are added with agitation to 7.5 ml of the **FFa** synthesized in example 2. After the complete dissolution of the polymer, 111.60 mg PEGA₂₀₀, previously purified (Mn: 248, 0.45 mmol), and 86.43 mg MeFlu PEGA₂₀₀ (0.15 mmol, Mn: 576.20 Da) are added. The pH of the obtained suspension of nanoparticles is increased slowly by adding a solution of NaOH 0.5M drop by drop and with agitation. After pH: 8.32 is reached, the suspension is subjected to ultrasound for 4 minutes, it is stirred at room temperature for 60 hours, it is again subjected to ultrasound for 4 minutes and is adjusted to pH: 7.43 by adding Na₂PHO₄ 0.1M. Finally, the suspension is filtered through a filter of a membrane Millex®-GS MF (Millipore) of 0.22 µm in order to obtain a transparent suspension denominated as **FFa@PVAm@MeFluPEGA.** The transmission electronic microscopy images of the obtained suspension are shown in figure 6. Groupings of several dozens of particles, which are probably generated by their encapsulation by the molecules of the polyvinylamine polymer, have been observed. The average diameter of the nanoparticles of iron oxide is 5.9 nm and the standard deviation is ±1.3 nm. An analysis by means of electron diffraction produces patterns which are compatible with the structure of the maghemite (γ-Fe₂O₃).

### Superparamagnetic properties of the ferrofluid FFa@PVAm@MeFluPEG-A

The variation with the temperature of the magnetic susceptibility ac in alternate magnetic field of the obtained sample was measured in a SQUID susceptometer from "Quantum Design" in comparison to the one of the acid suspension of nanoparticles prior to the coating. The obtained graphics are shown in figure 7. It can be observed that in the two suspensions there are some peaks, which vary with the alternation frequency of the field, in the susceptibility in phase χ' and outside of phase χ", which correspond to a superparamagnetic behavior. The temperature of the maximum at 117 Hz, which is usually called blocking temperature, is 30 K in both cases, in order words it does not vary in the process of the coating. This blocking temperature is in the range of the expected one for particles of this size (5.9 nm). However, it is observed that with the coating the susceptibility peak outside of the phase becomes narrower and more uniform and the intensity increases. This means that the suspension of coated nanoparticles has improved superparamagnetic properties compared to the original at the time of its use in applications such as the magnetothermia, which depend on the susceptibility outside of the phase.

### Application of the ferrofluid FFa@PVAm@MeFluPEGA in cellular studies

Cellular cultures of cells from the smooth muscles of the aorta of rats were prepared in the presence of multifunctional ferrofluid FFa@PVAm@MeFluPEGA. The cells were observed by means of fluorescence microscope Axiovert 200M equipped with Apotome, which provides a structured image using a 40x objective for immersion in oil. In these experiments, inks with blue emission for localization of the nuclei of the cells and lysotracker inks (Molecular Probes-Invitrogen) for marking the localization of the lysosomes are used. It is observed that the nanoparticles can be localized easily by their emission of green light. In this way, it was possible to follow-up the process of internalization of the particles in the cytoplasm of the cells and their introduction in the lysosomes. Figure 8 shows images of this process, which originally were taken in color but were converted in black and white in the figure. Figure 8a corresponds to a cell, into which a significant quantity of nanoparticles have penetrated; they can be distinguished as bright points which appear in green in the original image in colors. In the next image (Fig. 8b), the bright points, which are in red in the original image, show the localization of the lysosomes in the cellular cytoplasm, and the following image (Fig. 8c) shows a mixed image, in which the bright points are with a greater intensity than in the previous images, appear with yellow-orange color in the original image and coincide, with respect to the localization, with the previous images and result from the mixture of red and green emissions, which demonstrates the penetration of the nanoparticles into the lysosomes. Figure 9 shows the dispersion graphic, which presents the emission of channel 1, corresponding to the nanoparticles, along the axis x, and the emission of channel 2 corresponding to the emission of the marker of the lysosomes along the axis y. As it can be seen in the image, the two emissions show a total superposition, which demonstrates in unambiguous form the colocalization of the nanoparticles and the lysosomes.

## Claims

1. A method for obtaining coated and functionalized nanoparticles by means of Michael reaction, which comprises the steps:
a) reaction of at least one nanoparticle with a reagent, which comprises a Michael donor (B) or acceptor (C), and
b) reaction of the product obtained in (a) with a reagent, which comprises a Michael acceptor (C) or donor (B).
If in step (a) the reagent is a donor (B), in step (b) the reagent is an acceptor (C) or vice versa.

2. The method according to claim 1, wherein the nanoparticle is selected from metal, metal oxide or any combination thereof.

3. The method according to claim 2, wherein the metal oxide is iron oxide.

4. The method according to any of the claims 1 to 3, wherein the size of the nanoparticle is between 1 nm and 100 nm.

5. The method according to any of the claims 1 to 4, wherein the reagent of the step (a) comprises additionally a spacer R₁.

6. The method according to claim 5, wherein R₁ is an organic compound.

7. The method according to any of the claims 1 to 6, wherein the reagent of the step (b) comprises additionally a spacer R₂.

8. The method according to claim 7, wherein R₂ is an organic compound comprising groups solvatables in a liquid.

9. The method according to any of the claims 1 to 8, wherein the reagent of the step (a) comprises additionally a bonding group (A) selected from a cation, anion, alcoxylane precursor, thiol, alcohol, alcoxide, carboxylate, carboxylic anhydride, phosphate, polyelectrolyte, imine, nitrile, azide, amine, amide, phosphine or any combination thereof.

10. The method according to claim 9, wherein the anion is selected from a carboxylate, sulfate, phosphate group or any combination thereof.

11. The method according to any of the claims 1 to 10, wherein the Michael donor reagent (B) is selected from the list which comprises: β-diketone, malonic ester, β-ketoester, β-ketonotrile, nitro, amino, malonic acid, enamine, primary amine, secondary amine, tertiary amine, imine, hydrazine, guanadine, alcohol, thiol, phosphine, methylene, carbinol, organometallic compound, halide and any combination thereof.

12. The method according to any of the claims 1 to 11, wherein the Michael acceptor reagent (C) is selected from the list which comprises: conjugated enone, carbonyl, cyan, carboxylic, carboxylate, sulfonil, aldehyde, ester, nitrile, unsaturated nitro, carboxylate, acrylate, acrylamide, methacrylate, methacrylamide, acrylonitrile, vinyl ketone, vinyl sulfone and any combination thereof.

13. The method according to any of the claims 1 to 12, wherein the reagent of the step (b) comprises additionally at least one organic or inorganic residue (D) with physical, chemical or biological functionality.

14. The method according to any of the claims 1 to 13, wherein the organic or inorganic residue (D) with physical functionality is selected from fluoresceine, rodamine, rare earths complex, quantum dots or any combination thereof.

15. The method according to any of the claims 1 to 13, wherein the organic or inorganic residue (D) with chemical functionality is selected from a catalyst or an absorbent.

16. The method according to any of the claims 1 to 13, wherein the organic or inorganic residue (D) with biological functionality is selected from a compound with catalytic capacity, with recognizing capacity, with curative capacity or any combination thereof.

17. The method according to claim 16, wherein the compound with catalytic capacity is an enzyme.

18. The method according to claim 16, wherein the compound with recognizing capacity is selected from antibodies, oligonucleotides or any combination thereof.

19. The method according to claim 16, wherein the compound with curative capacity is a medicine.

20. The method according to any of the claims 1 to 19, wherein the steps (a) and/or (b) are carried out under conditions of temperature between 10 and 70° C.

21. A coated and functionalized nanoparticle obtainable by the method described according to any of the claims 1 to 20.

22. The use of the nanoparticles described according to claim 21 for the manufacturing of products selected from the list comprising cosmetics, paints, inks, catalysis, purification of waters, and biomedicine.

23. A dispersion of the coated and functionalized nanoparticles according to claim 21 in a liquid medium.

24. The dispersion according to claim 23, wherein the liquid medium of dispersion is selected from aqueous media, organic media or any combination thereof.

25. The use of the dispersion described in any of the claims 23 or 24 for the manufacturing of products selected from the list comprising cosmetics, paints, inks, catalysis, purification of waters, and biomedicine.
